Europäisches Patentamt

European Patent Office

Office européen des brevets

(11) Numéro de publication : **0 447 287 B1**

(12) **FASCICULE DE BREVET EUROPEEN**

(45) Date de publication du fascicule du brevet :
**11.08.93 Bulletin 93/32**

(21) Numéro de dépôt : **91400549.1**

(22) Date de dépôt : **28.02.91**

(51) Int. Cl.⁵ : **A61K 7/035**, A61K 7/00, A61K 7/48

(54) Utilisation en association, dans la préparation de compositions cosmétiques sous forme de poudres compactées, de microsphères creuses en matériau synthétique thermoplastique, de nitrure de bore hexagonal, et de N-acyl lysine.

(30) Priorité : **28.02.90 FR 9002508**

(43) Date de publication de la demande :
**18.09.91 Bulletin 91/38**

(45) Mention de la délivrance du brevet :
**11.08.93 Bulletin 93/32**

(84) Etats contractants désignés :
**AT BE CH DE DK ES FR GB GR IT LI NL SE**

(56) Documents cités :
**EP-A- 0 139 481
EP-A- 0 254 612
STN SERVEUR DE BASES DE DONNEES, Karsruhe, DE, FICHIER CHEMICAL ABSTRACTS, vol. 109, no. 18, résumé no. 155980n, Columbus, Ohio, US; & JP-A-63 119 411 (SHOWA) 24-05-1988
PATENT ABSTRACTS OF JAPAN, vol. 12, no. 37 (C-473)[2884], 4 février 1988; & JP-A-62 187 405 (NONOGAWA SHOJI K.K.) 15-08-1987**

(73) Titulaire : **L'OREAL
14, Rue Royale
F-75008 Paris (FR)**

(72) Inventeur : **Farer, Alan M.
14, Bromley Court
Marlboro, New Jersey 07751 (US)**
Inventeur : **Hanna, Fifi
642, Devon Street
Kearny, New Jersey 07032 (US)**
Inventeur : **Fox, Elisa L.
1231 Valley Road
Upper Montclair, New Jersey 07043 (US)**
Inventeur : **Penicnak, John A.
151 Lookout Road
Mountain Lakes, New Jersey 07046 (US)**

(74) Mandataire : **Tonnellier, Jean-Claude et al
Cabinet Nony & Cie. 29, rue Cambacérès
F-75008 Paris (FR)**

## Description

L'invention a pour objet l'utilisation, dans la préparation d'une composition cosmétique sous forme de poudre compactée, d'une association ternaire favorisant l'homogénéisation des ingrédients de la composition. Il s'agit de l'utilisation en association, d'un matériau synthétique thermoplastique sous forme de microsphères creuses, de particules de nitrure de bore hexagonal et d'un dérivé acylé de la lysine.

On sait que les compositions cosmétiques actuelles sous forme de poudres comportent de nombreux ingrédients, et leur fabrication nécessite des opérations de mélangeage, de broyage et de tamisage qui sont relativement longues et coûteuses en énergie.

Ces opérations sont encore plus délicates dans la préparation de poudre compactée, qui comporte généralement l'addition d'une phase huileuse, par exemple par pulvérisation, sur le mélange des particules des autres ingrédients. La réalisation d'une répartition aussi uniforme que possible de la phase huileuse, et l'homogénéisation finale du produit après addition de la phase huileuse, sont particulièrement longues et difficiles à mettre en oeuvre.

La présente invention a pour but de remédier à ces inconvénients, grâce à l'utilisation, en association, de microsphères creuses en matériau synthétique thermoplastique, de particules de nitrure de bore hexagonal et d'un dérivé N-acylé de la lysine. Une telle association confère à la masse des particules, lors de la préparation, une fluidité exceptionnelle et permet donc de réduire les coûts de fabrication en temps et en énergie. En outre cette association permet d'obtenir des poudres compactées de bonne qualité, ayant des propriétés de compacité et de résistance aux chocs satisfaisantes tout en étant d'application agréable.

L'utilisation de microsphères creuses d'un matériau synthétique thermoplastique dans des poudres libres non compactées pour le maquillage et les soins de la peau a déjà été décrite, notamment dans le brevet français 2600532. L'utilisation de microsphères creuses dans des compositions cosmétiques sous forme de poudre a également été décrite dans la demande de brevet japonais 60-18404. Les microsphères creuses utilisées concrètement selon cette demande de brevet japonais, ont une masse spécifique relativement élevée, et ne conviennent pas pour la mise en oeuvre de la présente invention.

L'utilisation de particules de nitrure de bore hexagonal dans des compositions cosmétiques sous forme de poudres a été décrite dans la demande de brevet japonais 62-49247.

L'utilisation de talc revêtu d'un acylamino acide dans une composition cosmétique sous forme de poudre contenant de la silice, du mica, du polyéthylène et un liant particulier comprenant une combinaison de polydiméthylsiloxane de malate de distéaryle et de pentahydrosqualène, a été décrite dans le brevet US 4 837 011.

On a maintenant découvert que l'association des trois ingrédients mentionnés ci-dessus confère au mélange de particules utilisé dans la préparation d'une poudre compactée une fluidité bien supérieure à celle obtenue avec les ingrédients utilisés jusqu'à présent.

La présente invention a donc pour objet l'utilisation, dans la préparation d'une composition cosmétique sous forme de poudre compactée, de 1 à 55 % en poids, par rapport au poids total de la composition, d'une association de trois constituants favorisant l'homogénéisation des ingrédients de la composition, à savoir :
- x % en poids de microsphères creuses en matériau synthétique thermoplastique ayant une masse spécifique inférieure à 0,1g/cm$^3$ et ayant des dimensions inférieures à 30 µm,
- y % en poids de particules de nitrure de bore hexagonal,
- z % en poids de particules de N-acyl lysine,

x, y, z étant des nombres pouvant varier respectivement dans les gammes suivantes :
    x : 0,02-30
    y : 0,2-90
    z : 8 à 99,78
étant entendu que x + y + z = 100.

Il faut noter que les proportions x, y et z sont les proportions relatives des trois constituants de l'association, et non les proportions de ces trois constituants dans la composition cosmétique qui contient bien entendu, outre les trois produits en association, les divers ingrédients habituellement présents dans les compositions sous forme de poudres compactées.

Dans des modes de réalisation particuliers, l'association mentionnée peut encore présenter les caractéristiques suivantes, prises isolément ou en combinaison :
- x est un nombre pouvant varier de 0,5 à 2 ;
- y est un nombre pouvant varier de 5 à 25 ;
- z est un nombre pouvant varier de 75 à 95 ;
- les microsphères creuses ont des dimensions de 10 à 20 µm environ.

Les trois constituants de l'association définie ci-dessus représentent de préférence de 10 à 35 % en poids du poids total de la composition cosmétique.

Les microsphères creuses sont préparées selon des procédés connus, tels que ceux décrits dans le brevet US 3 615 972 et la demande de brevet européen n° 0 056 219.

La partie creuse des microsphères contient un gaz tel qu'un hydrocarbure (butane, pentane), de l'air ou tout autre gaz utilisé classiquement.

Ces microsphères peuvent être réalisées en tous matériaux thermoplastiques non toxiques et non irritants. Ces matériaux peuvent être par exemple des polymères ou copolymères de dérivés éthyléniques (par exemple polyéthylène, polystyrène, copolymère chlorure de vinyle-acrylonitrile, etc...) des polyamides, des polyesters, des polymères urée-formaldéhyde, des copolymères de chlorure de vinylidène (par exemple chlorure de vinylidène-acrylonitrile), etc...

La masse spécifique des microsphères creuses est de préférence de l'ordre de 0,01 à 0,1g/cm³.

Les particules de nitrure de bore hexagonal ont de préférence des dimensions pouvant aller de 0,1 à 30 µm.

Parmi les microsphères creuses utilisables dans les compositions de l'invention, on citera notamment celles qui sont commercialisées sous les dénominations EXPANCEL 551 DE 20 (dimensions : 20 µm) et 551 DE 12 (dimensions : 12 µm) par la Société Kemanord Plast.

Les particules de N-acyl lysine ont de préférence des dimensions de 5 à 30 µm. Parmi les dérivés N-acylés de la lysine, on citera en particulier ceux dont le groupement acyle est dérivé d'un acide gras ayant 8 à 18 atomes de carbone. Parmi les dérivés N-acylés préférés on citera notamment la N-lauroyl lysine.

L'invention a également pour objet une composition cosmétique sous forme de poudre compactée contenant de 1 à 55 % en poids, et de préférence de 10 à 35 % en poids, de l'association ternaire telle que définie précédemment.

La composition de l'invention contient en outre des charges et/ou des pigments usuels, ainsi qu'une phase huileuse.

Les pigments et/ou charges sont ceux habituellement utilisés dans les compositions cosmétiques sous forme de poudres compactées. Les pigments sont choisis notamment parmi les pigments minéraux, les pigments organiques et les pigments nacrés.

Parmi les pigments minéraux, on peut citer, à titre d'exemples :
- le dioxyde de titane (rutile ou anatase), éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome (CI 77288) ;
- l'oxyde de chrome hydraté ; et
- le bleu ferrique (CI 77510).

Parmi les pigments organiques, on peut citer, en particulier, les pigments :
D & C red n° 19 (CI 45170) ;
D & C red n° 9 (CI 15585) ;
D & C red n° 21 (CI 45380) ;
D & C orange n° 4 (CI 15510) ;
D & C orange n° 5 (CI 45370) ;
D & C red n° 27 (CI 45410) ;
D & C red n° 13 (CI 15630) ;
D & C red n° 7 (CI 15850:1) ;
D & C red n° 6 (CI 15850:2) ;
D & C yellow n° 5 (CI 19140) ;
D & C red n° 36 (CI 12085) ;
D & C orange n°10 (CI 45425) ;
D & C yellow n° 6 (CI 15985) ;
D & C red n° 30 (CI 73360) ;
D & C red n° 3 (CI 45430) ;
le noir de carbone (CI 77266) ; et
les laques à base de carmin de cochenille (CI 75470).

Les pigments nacrés peuvent être choisis notamment parmi :
- les pigments nacrés blancs, tels que le mica recouvert d'oxyde de titane, l'oxychlorure de bismuth ; et
- les pigments nacrés colorés, tel que le mica titane avec des oxydes de fer, le mica titane avec du bleu ferrique ou de l'oxyde de chrome, le mica titane avec un pigment organique du type précité, ainsi que ceux à base d'oxychlorure de bismuth.

Ces pigments peuvent représenter, de préférence, de 0,1 à 2 % en poids du poids total de la composition
Les charges sont choisies notamment parmi :

- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoelithe, lipidolithe, biotite) ou d'origine synthétique. Les micas sont généralement transparents et permettent de conférer à la peau un aspect satiné ; Généralement, dans les compositions de l'invention, les micas représentent de 20 à 70 % et, de préférence, de 30 à 50 %, du poids total de la composition ;

- le carbonate ou l'hydrocarbonate de magnésium, qui possèdent notamment des propriétés de fixation des parfums ; ils peuvent être présents, dans la composition de l'invention, à raison de 1 à 10 % et de préférence de 2 à 7 % du poids total de la composition ;

- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, etc... Ces savons, présents généralement sous la forme de particules ayant des dimensions inférieures à 10 µm, ont un toucher onctueux et facilitent l'adhérence de la poudre sur la peau ; Ces savons métalliques sont généralement présents à raison de 1 à 10 % et de préférence de 2 à 7 % du poids total de la composition ;

- les poudres de polymères synthétiques, tels que le polyéthylène, les polyesters (par exemple isophtalate ou téréphtalate de polyéthylène), et les polyamides (par exemple le nylon), sous la forme de particules ayant des dimensions inférieures à 50 µm, qui possèdent des propriétés absorbantes et permettent de conférer à la peau un aspect velouté ; ces poudres de polymères synthétiques sont présentes dans la composition de l'invention à raison de 1 à 40 % en poids et de préférence de 10 à 30 % en poids, par rapport au poids total de la composition.

La composition de l'invention peut également contenir d'autres charges telles que le talc, l'amidon, le kaolin, les oxydes de zinc et de titane, le carbonate de calcium précipité, etc.

La phase grasse comprend généralement des corps gras liquides à la température ambiante qui sont présents dans la composition à raison de 2 à 10 % en poids et de préférence de 4 à 8 % en poids.

Parmi les corps gras liquides à température ambiante, on peut citer les huiles minérales, animales, végétales ou synthétiques, ou encore les huiles de silicone. Il s'agit par exemple des huiles suivantes : l'huile de vaseline, la lanoline liquide, l'huile d'arara, les huiles de sésame, de macadamia ou de jojoba, et les triglycérides de synthèse.

La phase grasse peut également contenir un polymère synthétique oléosolubles dont l'utilisation dans les cosmétiques est connues.

Parmi les polymères synthétiques oléosolubles, on peut citer les copolymères polyvinylpyrrolidone/hexadécène ou PVP/eicosène tels que les produits vendus par GAF Corp. sous la marque commerciale GANEX V-216 et GANEX V-220.

La phase grasse éventuellement ajoutée représente 0 à 25 % du poids total de la composition, et de préférence 3 à 20 %.

D'autres ingrédients divers peuvent être introduits dans les compositions à compacter, tels que des antiseptiques (trichloro diphényl éther, agents cationiques, acide borique, etc...) qui sont utilisés notamment dans les poudres désodorisantes pour le corps ou les pieds et dans les poudres pour bébés ; des agents astringents, qui sont utilisés dans les poudres désodorisantes ou dans les poudres pour les pieds, tels que l'hydroxychlorure d'aluminium ou les aluns ; des filtres solaires ; des agents adoucissants ; des agents hydratants (sorbitol, glycérine) ; des agents cicatrisants ; des agents anti-radicaux libres ; des vitamines ; des parfums ; etc...

Ces ingrédients peuvent représenter jusqu'à 5 % du poids total de la composition.

Enfin, les poudres compactées selon l'invention contiennent éventuellement des agents de consistance hydrosolubles (jusqu'à 20 % en poids), tels que des gommes naturelles ou synthétiques, des dérivés de cellulose ou des polymères acryliques.

Les poudres de l'invention sont préparées selon les techniques classique de préparation des poudres compactées. Généralement, on opère de la façon suivante : on mélange et homogénéise les constituants particulaires définis ci-dessus, on prépare par ailleurs la phase huileuse, contenant les agents de conservation et éventuellement le parfum, on pulvérise la phase huileuse sur la phase particulaire, tout en mélangeant ; on répartit la poudre résultante dans des conteneurs appropriés et l'on presse la poudre de façon usuelle à une pression suffisante pour obtenir une poudre compacte ayant le degré de compacité désiré.

L'exemple suivant illustre l'invention sans toutefois la limiter.

EXEMPLE:

On a préparé une poudre compactée ayant la composition suivante :

| Ingrédients particulaires | % en poids |
|---|---|
| Mica (Séricite S152 ; Presperse, Inc.)............ | 44 |
| Poudre de nylon (Orgasol 2002D-Extra ; Lipo Chemical, Inc.)................. | 25 |
| N-lauroyl lysine (Amihope LL ; Centerchem)........ | 15 |
| Stéarate de zinc................................. | 1 |
| Carbonate de magnésium............................ | 2 |
| Nitrure de bore hexagonal......................... | 3 |
| Microsphères creuses (Expancel 551DE20)........... | 0,25 |
| Pigments.......................................... | 1,5 |
| **Phase huileuse** | |
| Stéarate d'octyldodécyle* | |
| Maleate de dioctyle ...................... | 8 |
| Malate de diisostéaryle | |
| Conservateurs.................................... | 0,125 |
| Parfum........................................... | 0,125 |
| TOTAL : | 100,00 |

\* Ceraphyl 847, Malinckrödt Inc.

**Revendications**

1. Utilisation, dans la préparation d'une composition cosmétique sous forme de poudre compactée, de 1 à 55 % en poids, par rapport au poids total de la composition, d'une association de trois constituants favorisant l'homogénéisation des ingrédients de la composition, à savoir :
   - x % en poids de microsphères creuses en matériau synthétique thermoplastique ayant une masse spécifique inférieure à 0,1g/cm$^3$ et ayant des dimensions inférieures à 30 μm,
   - y % en poids de particules de nitrure de bore hexagonal,
   - z % en poids de particules de N-acyl lysine,
   x, y, z étant des nombres pouvant varier respectivement dans les gammes suivantes :
   x : 0,02-30
   y : 0,2-90
   z : 8 à 99,78
   étant entendu que x + y + z = 100

2. Utilisation selon la revendication 1, caractérisée par le fait que x est un nombre pouvant varier de 0,5 à 2.

3. Utilisation selon l'une quelconque des revendications précédentes, caractérisée par le fait que y est un nombre pouvant varier de 5 à 25.

4. Utilisation selon l'une quelconque des revendications précédentes, caractérisée par le fait que z est un nombre pouvant varier de 75 à 95.

5. Utilisation selon l'une quelconque des revendications précédentes, caractérisée par le fait que l'ensemble des constituants de ladite association représente de 10 à 35 % en poids du poids total de la composition.

6. Utilisation selon l'une quelconque des revendications précédentes, caractérisée par le fait que les dites microsphères creuses sont réalisées en un matériau choisi parmi les polymères ou copolymères dérivés d'hydrocarbures éthyléniques, des polyesters, des polyamides, des polymères urée-formaldéhydes et des copolymères de chlorure de vinylidène.

7. Utilisation selon l'une quelconque des revenedications précédentes, caractérisée par le fait que l'acyle de ladite N-acyle lysine est dérivé d'un acide gras ayant 8 à 18 atomes de carbone.

8. Utilisation selon la revendication 7, caractérisée par le fait que ledit acyle est une groupement lauroyle.

9. Composition cosmétique sous forme de poudre compactée caractérisée par le fait qu'elle contient de 1 à 55 % en poids de l'association de trois constituants telle que définie dans l'une quelconque des revendications précédentes.

10. Composition selon la revendication 9, caractérisée par le fait qu'elle contient de 10 à 35 % de l'ensemble des trois constituants de ladite association.


**Patentansprüche**

1. Verwendung, zur Herstellung einer kosmetischen Zubereitung in Form von Kompaktpuder, von 1 bis 55 Gew.-%, bezogen auf das Gesamtgewicht der Zubereitung, einer Kombination von drei Bestandteilen, welche die Homogenisierung der Komponenten der Zubereitung fördern, von:
   - x Gew.-% Mikrohohlkugeln aus thermoplastischem, synthetischem Material mit einem spezifischen Gewicht von weniger als $0,1$ $g/cm^3$ und Größen von weniger als 30 $\mu m$,
   - y Gew.-% hexogonaler Bornidridpartikel,
   - z Gew.-% N-Acyllysinpartikel, wobei die Zahlen
   x, y, z innerhalb folgender Bereiche variieren können:
       x: 0,02-30
       y: 0,2-90
       z: 8 bis 99,78
   und x + y + z = 100 ausmachen.

2. Verwendung nach Anspruch 1, dadurch gekennzeichnet, daß x eine Zahl bedeutet, die von 0,5 bis 2 variieren kann.

3. Verwendung nach einem der vorstehenden Ansprüche, dadurch gekennzeichnet, daß y eine Zahl bedeutet, die von 5 bis 22 variieren kann.

4. Verwendung nach einem der vorstehenden Ansprüche, dadurch gekennzeichnet, daß z eine Zahl bedeutet, die von 75 bis 95 variieren kann.

5. Verwendung nach einem der vorstehenden Ansprüche, dadurch gekennzeichnet, daß die Gesamtheit der Bestandteile der Kombination 10 bis 35 Gew.-% des Gesamtgewichtes der Zubereitung ausmacht.

6. Verwendung nach einem der vorstehenden Ansprüche, dadurch gekennzeichnet, daß die Mikrohohlkugeln aus einem Material hergestellt sind, das aus polymeren oder copolymeren ethylenischen Kohlenwasserstoffderivaten, Polyestern, Polyamiden, Harnstofformaldehydpolymeren und Copolymeren von Vinylidenchloride ausgewählt wird.

7. Verwendung nach einem der vorstehenden Ansprüche, dadurch gekennzeichnet, daß die Acylgruppe des N-Acyllsins von einer Fettsäure mit 8 bis 18 Kohlenstoffatomen stammt.

8. Verwendung nach Anspruch 7, dadurch gekennzeichnet, daß die Acylgruppe eine Lauroylgruppe ist.

9. Kosmetische Zubereitung in Form von Kompaktpuder, dadurch gekennzeichnet, daß sie 1 bis 55 Gew.-% einer Kombination von drei Bestandteilen gemäß einem der vorstehenden Ansprüche enthält.

10. Zubereitung nach Anspruch 9, dadurch gekennzeichnet, daß sie 10 bis 35 Gew.-% aller drei Bestandteile der Kombination enthält.

**Claims**

1. Use, in the preparation of a cosmetic composition in the form of a compacted powder, of 1 to 55% by weight, relative to the total weight of the composition, of a combination of three constituents which promote homogenisation of the ingredients of the composition, namely:
   - x % by weight of hollow microspheres made of thermoplastic synthetic material having a density of less than 0.1 g/cm$^3$ and which are smaller than 30 $\mu$m in size,
   - y % by weight of hexagonal boron nitride particles,
   - z % by weight of N-acyllysine particles,
   x, y and z being numbers which can vary, respectively, within the following ranges:
   x : 0.02-30
   y : 0.2-90
   z : 8 to 99.78
   on the understanding that x + y + z = 100.

2. Use according to Claim 1, characterised in that x is a number which can vary from 0.5 to 2.

3. Use according to either of the preceding claims, characterised in that y is a number which can vary from 5 to 25.

4. Use according to any one of the preceding claims, characterised in that z is a number which can vary from 75 to 95.

5. Use according to any one of the preceding claims, characterised in that the constituents of the said combination, taken together, represent from 10 to 35% by weight of the total weight of the composition.

6. Use according to any of the proceding claims, characterised in that the said hollow microspheres are made from a material chosen from the polymers or copolymers derived from ethylenic hydrocarbons, from polyesters, from polyamides, from urea-formaldehyde polymers and from vinylidene chloride copolymers.

7. Use according to any one of the preceding claims, characterised in that the acyl radical of the said N-acyllysine is derived from a fatty acid having 8 to 18 carbon atoms.

8. Use according to Claim 7, characterised in that the said acyl radical is a lauroyl group.

9. Cosmetic composition in the form of a compacted powder, characterised in that it contains from 1 to 55% by weight of the combination of three constituents as is defined in any one of the preceding claims.

10. Composition according to Claim 9, characterised in that it contains from 10 to 35% of the three constituents of the said combination, taken together.